# DEMANDE DE BREVET EUROPEEN

(11) **EP 1 072 228 A1**
(43) Date de publication de la demande: **31.01.2001**
(21) Numéro de dépôt: 00450013.8
(22) Date de dépôt: 25.07.2000
(51) Int. Cl.: A61B 17/70

(54) **Dispositif de liaison intervertebrale implantable**

(30) Priorité: 27.07.1999 FR 9909981
(71) Demandeur: Société Etudes et Developpements S.E.D., 19130 Voutezac (FR); Multi-Poles Conseils, 87600 Rochechouart (FR)
(72) Inventeur: Jammet, Jean, 19130 Voutezac (FR); Lenfant, Jean-Pierre, 87600 Rochechouart (FR); Peyre, André, 33000 Bordeaux (FR); Jammet, David, 19130 Voutezac (FR)
(74) Mandataire: Thébault, Jean-Louis

(57) **Abrégé**

- L'objet de l'invention est un dispositif de liaison intervertébrale implantable comprenant au moins deux vis pédiculaires (1, 2) munies en partie supérieure d'une tête hexagonale (3) prolongée par une portée cylindrique (4, 4') et une liaison intervis de longueur réglable ancrée à chaque extrémité sur lesdites portées, caractérisé en ce que ladite liaison intervis est constituée, d'une part, d'une bille (7, 7') de préférence en un matériau déformable élastique enfilée sur chaque portée (4) et, d'autre part, de deux éléments de liaison (E1, E2 ; E'1, E'2) dans le prolongement l'un de l'autre et reliés entre eux par un système à vis (17, 18 ; 18', 41, 42) de réglage d'éloignement, les extrémités libres des éléments étant conformées en coupelle (8, 9 ; 8', 9') concave embrochable sur ladite portée (4, 4') et épousant ladite bille (7, 7') en sorte qu'au droit de chaque vis pédiculaire (1, 2 ; 1', 2') la bille soit prise en sandwich entre deux coupelles (8, 9 ; 8', 9') de deux liaisons associées à la vis, des moyens (6, 6') étant prévus pour rapprocher l'une vers l'autre les deux coupelles (8, 9 ; 8', 9') en serrant ladite bille (7, 7') et solidariser l'ensemble de la vis pédiculaire.
- Application aux traitements de la colonne vertébrale.

## Description

La présente invention a trait à un dispositif de liaison intervertébrale implantable plus particulièrement destiné à solidariser deux ou plusieurs vertèbres.

L'invention vise à replacer et maintenir selon une disposition aussi proche que possible de la normale les vertèbres déplacées ou déficientes, notamment dans le traitement de certains cas de scoliose, de cyphose, de lordose exagérée, d'instabilité vertébrale ou de diminution importante de l'espace intervertébral quelle qu'en soit la cause.

Par le document FR 2 698 533, on connaît un dispositif de liaison intervertébral implantable permettant une liaison deux à deux de plusieurs vertèbres à l'aide de vis pédiculaires prolongées par une portée cylindrique sur laquelle est enfilé un embout sphérique d'un élément de liaison intervis muni à son autre extrémité, soit d'un autre embout sphérique, soit d'une coupelle concavo-sphérique coopérant avec un embout sphérique d'une seconde liaison visant une même vis pédiculaire.

L'élément de liaison est du type à ridoir et la solidarisation de l'ensemble est assurée par des écrous engagés sur les extrémités des portées cylindriques et venant plaquer chaque embout ou chaque association embout-coupelle selon le cas, contre un siège sphérique ménagé sur une tête hexagonale de la vis pédiculaire.

Si un tel dispositif permet un réglage de l'écart intervertébral, aussi bien en distraction qu'en rétraction, par une action sur le ridoir de chaque liaison intervis, et s'il permet également une prise en compte du non parallélisme des axes des vis pédiculaires in situ par un ajustement de l'angle formé par deux liaisons partant d'une même vis pédiculaire, grâce au dispositif à rotule assurant la jonction entre vis et éléments de liaison intervis, cet ensemble de liaison intervertébral présente néanmoins de sérieux inconvénients tenant à sa rigidité interdisant au patient notamment toute flexion ou rotation du buste.

Par ailleurs, l'ensemble des composants du système étant en métal tel que par exemple un alliage de titane, il est difficile de conserver un serrage efficace des éléments de la rotule de liaison et souvent une intervention postérieure à la mise en place de la prothèse intervertébrale sera nécessaire pour resserrer les écrous de blocage desdites rotules.

La présente invention vise à pallier ces divers inconvénients en proposant un dispositif de liaison intervertébral implantable, apte non seulement à permettre un réglage d'écart intervertébral et une adaptation à l'angulation des pédicules l'un par rapport à l'autre, mais procurant en outre au patient un meilleur confort par une conception du dispositif lui conférant une élasticité propre à lui permettre des mouvements de flexion, rotation du buste et amortissant les chocs.

A cet effet, l'invention a pour objet un dispositif de liaison intervertébrale implantable comprenant au moins deux vis pédiculaires munies en partie supérieure d'une tête hexagonale prolongée par une portée cylindrique et une liaison intervis de longueur réglable ancrée à chaque extrémité sur lesdites portées, caractérisé en ce que ladite liaison intervis est constituée, d'une part, d'une bille enfilée sur chaque portée et, d'autre part, de deux éléments de liaison dans le prolongement l'un de l'autre et reliés entre eux par un système à vis de réglage d'éloignement, les extrémités libres des éléments étant conformées en coupelle concave embrochable sur ladite portée et épousant ladite bille en sorte qu'au droit de chaque vis pédiculaire la bille soit prise en sandwich entre deux coupelles de deux liaisons associées à la vis, des moyens étant prévus pour rapprocher l'une vers l'autre les deux coupelles en serrant ladite bille et solidariser l'ensemble de la vis pédiculaire.

De préférence, la bille est en un matériau déformable élastique.

La nature élastique de la jonction à rotule au niveau de chaque vis pédiculaire permet un amortissement des chocs et une meilleure répartition des contraintes le long de la prothèse.

Celle-ci n'étant pas constituée par une chaîne rigide mais par un chapelet d'éléments ou maillons tous susceptibles à chacune de leurs extrémités d'un certain débattement élastique dans toutes les directions, y compris axialement aux vis pédiculaires, le patient a ainsi la possibilité de mouvoir son buste en flexion et rotation par rapport au bassin, d'une certaine amplitude, en ménageant d'autant le rachis.

Avantageusement, le système à vis reliant les deux éléments d'une liaison intervis est constitué d'un manchon taraudé solidaire de l'un des éléments et d'une tige filetée coopérante, reliée à l'autre élément par l'intermédiaire d'un système de blocage à butée élastique.

Suivant une variante de réalisation, le système à vis reliant les deux éléments d'une liaison intervis est constitué d'une tige munie, à une extrémité, d'une tête filetée engagée dans un taraudage d'un manchon reçu dans l'un des deux éléments susdits et à son autre extrémité, d'une tête cylindrique reçue dans l'autre des éléments, des moyens étant prévus, d'une part, pour permettre soit la libre rotation, soit le blocage en rotation dudit manchon dans l'élément porteur et, d'autre part, pour retenir et bloquer en rotation ladite tête cylindrique dans l'élément porteur, une butée élastique étant prévue entre la tête et l'élément.

Un tel dispositif non seulement permet le réglage de l'écart intervertébral aussi bien en distraction qu'en rétraction, mais assure une liaison amortisseuse de chocs entre les deux éléments de la liaison tout en bloquant en rotation la partie vis, garantissant ainsi l'écart désiré.

Aux deux extrémités du dispositif de liaison intervertébrale, les moyens de contention-serrage de la bille des deux vis pédiculaires extrêmes peuvent être allégés, puisqu'il n'y a pas de départ d'une autre liaison, en prévoyant la prise en sandwich des billes en question entre la coupelle de l'unique élément de liaison et une coupelle simple faisant office de rondelle interposée entre la bille et la tête hexagonale de la vis pédiculaire ou l'écrou de blocage de l'ensemble, selon le cas.

Bien entendu les coupelles présentent un trou de passage de la portée cylindrique des vis pédiculaires de diamètre sensiblement supérieur à celui de ladite portée, afin d'assurer auxdites coupelles un certain débattement angulaire omnidirectionnel, cependant que les faces, respectivement de la tête hexagonale et de l'écrou de blocage de l'ensemble, tournées vers la bille sont configurées en correspondance avec la face en regard des coupelles.

La bille est de préférence sphérique, mais peut éventuellement avoir une autre forme appropriée, cependant que les faces des coupelles tournées vers la bille sont soit sphériques, soit tronconiques, soit encore à facettes.

D'autres caractéristiques et avantages ressortiront de la description qui va suivre d'un mode de réalisation préféré du dispositif de l'invention, description donnée à titre d'exemple uniquement et en regard des dessins annexés sur lesquels :
- figure 1 est une vue en élévation latérale d'un dispositif de liaison de vertèbres conforme à l'invention ;
- figure 2 est une vue en éclaté d'une vis pédiculaire du dispositif de la figure 1, avec ses deux éléments de liaison ;
- figure 3 illustre les possibilités de débattement angulaire d'un dispositif du type de la figure 2, une fois monté ;
- figure 4 est une vue partielle agrandie de la liaison entre les deux vis de la figure 1 ;
- figure 5 est une vue en coupe axiale d'une variante de réalisation d'un élément de liaison ;
- figure 6 est une vue en éclaté d'une variante de réalisation du dispositif de liaison selon l'invention ;

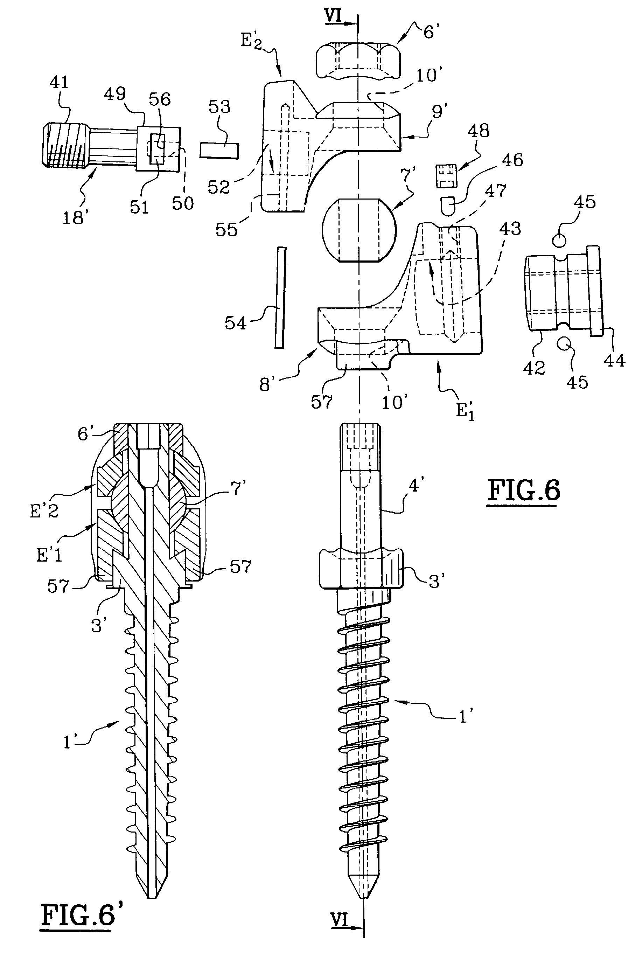

- figure 6' représente l'ensemble de la figure 6 monté, en coupe suivant la ligne VI-VI ;

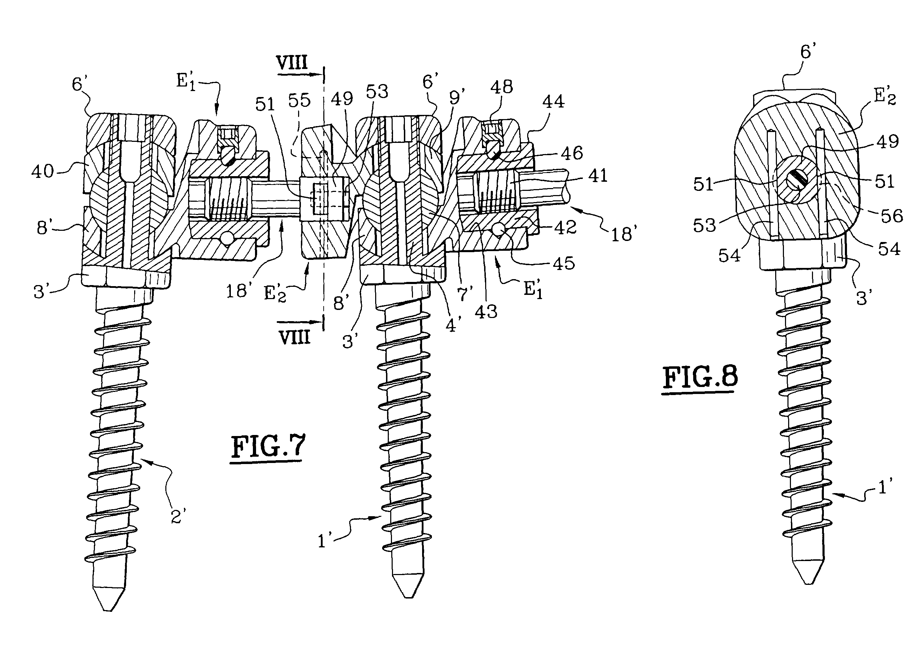

- figure 7 illustre l'extrémité d'un dispositif de liaison mettant en oeuvre la variante de la figure 6, et
- figure 8 est une vue en coupe suivant la ligne VIII-VIII du dispositif de la figure 7.

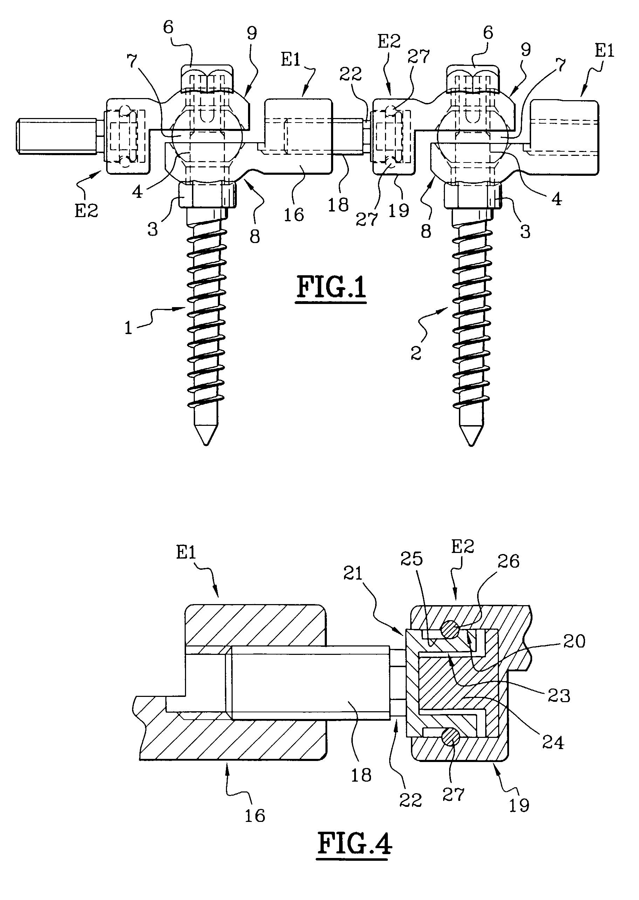

Sur la figure 1, on a représenté la liaison entre deux vis pédiculaires 1 et 2 par un dispositif conforme à l'invention.

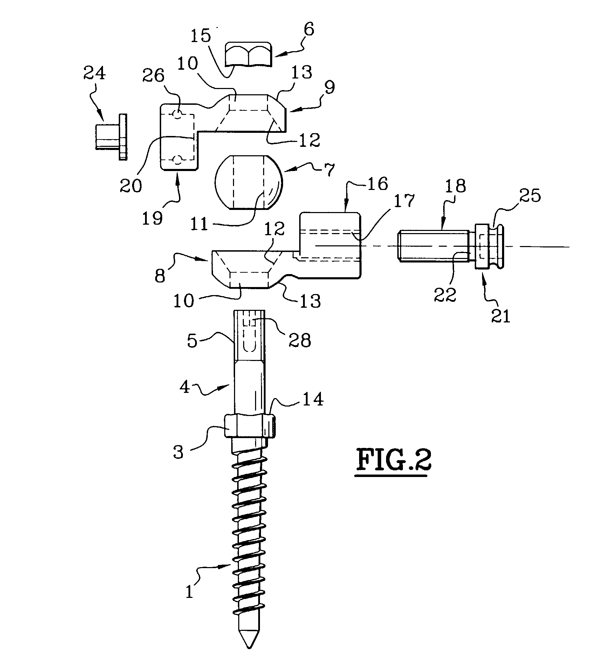

Les vis 1, 2 comportent à leur extrémité supérieure une tête hexagonale 3 conventionnelle prolongée par une portée cylindrique 4 filetée à son extrémité en 5 (figure 2) afin de recevoir un écrou 6 de blocage d'une bille 7 faisant office de rotule, elle-même prise en sandwich entre une coupelle inférieure 8 et une coupelle supérieure 9, toutes les deux munies en leur centre d'un perçage 10 de diamètre sensiblement supérieur à celui de la portée cylindrique 4, afin de permettre le libre enfilage des coupelles sur la portée ainsi qu'un certain débattement radialement à la portée.

La bille 7 est munie d'un alésage 11 de diamètre correspondant à celui de la portée 4 sur laquelle elle est également librement enfilée.

La bille 7 est par exemple sphérique et est réalisée de préférence en un matériau compressible élastiquement, tel qu'une matière plastique, par exemple du polyéthylène ou du polyuréthanne.

Les coupelles 8, 9 sont de forme générale concave avec une face 12 tournée vers la bille 7 de forme sphérique correspondant à la bille ou de forme tronconique comme illustré sur la figure 2. Eventuellement, la face 12 peut être à facettes, au nombre de 6 ou 8 par exemple.

La face externe 13 des coupelles est par exemple convexo-sphérique en sorte d'épouser respectivement une face concave correspondante conformée soit (14) sur la tête hexagonale 3, soit (15) sur l'écrou de blocage 6.

Chacune des deux vis 1, 2 étant reliée à une autre vis pédiculaire comporte une coupelle inférieure 8 faisant partie d'une première liaison avec une vis et une coupelle supérieure 9 faisant partie d'une seconde liaison avec une autre vis.

Chaque liaison intervis comprend ainsi deux éléments référencés d'une manière générale en E1 et E2 respectivement, qui peuvent être rapprochés ou éloignés grâce à un système à vis assurant leur jonction dans le prolongement l'un de l'autre.

A cet effet, l'élément E1 comprend un bloc 16 solidaire de la coupelle inférieure 8 latéralement et muni d'un perçage taraudé 17 susceptible de recevoir une tige filetée 18 solidaire de l'élément E2.

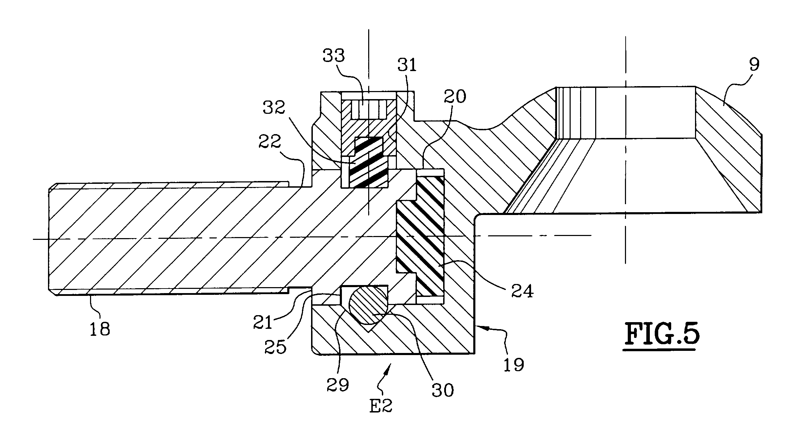

L'élément E2 comprend un bloc 19 solidaire de la coupelle supérieure 9 latéralement et muni d'un logement cylindrique 20 (figure 4) susceptible d'accueillir une tête 21 de forme générale cylindrique ménagée à l'extrémité de la tige filetée 18.

Bien entendu les positions des éléments E1, E2 peuvent être inversées, la coupelle 8 de l'élément E1 pouvant être placée en position de coupelle supérieure et inversement pour la coupelle 9 de l'élément E2.

La tête 21 est reliée à la tige filetée 18 par un cou 22 de section hexagonale pour permettre la rotation de la vis 18.

La tête 21 est par ailleurs creuse et comporte un logement cylindrique 23 axial s'ouvrant en direction du fond du logement 20.

Un bloc-tampon 24 en matériau compressible élastique, par exemple du même matériau que la bille 7, est reçu dans le logement 20 et occupe le logement 23 de la tête 21.

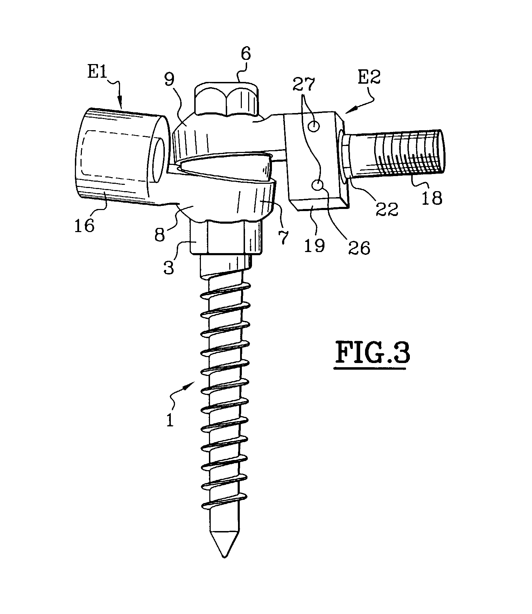

La face périphérique externe de la tête 21 est munie d'une gorge circulaire 25, cependant que le bloc 19 est percé latéralement (figure 3) de deux trous borgnes parallèles 26, à hauteur de la paroi du logement 20, afin d'y engager deux goupilles 27 de blocage de la tête 21.

La tête 21 est engagée dans le logement 20 en comprimant le bloc élastique 24.

Lorsque la gorge 25 est arrivée à hauteur des perçages 26, ce qui est contrôlé par l'affleurement de la face arrière de la tête 21 et de la face avant du bloc 19, les goupilles 27 sont insérées dans les perçages 26 et pénètrent dans la gorge 25, de part et d'autre de la tête 21. Cette dernière est ainsi retenue prisonnière et est freinée dans sa rotation du fait de la pression élastique du bloc-tampon 24.

Il est à noter que la liaison E1 E2 peut ainsi encaisser élastiquement des chocs en compression axiale, absorbés par le bloc 24.

Comme on peut l'observer sur la figure 3, la liaison à rotule assurée par la bille 7 prise en sandwich entre les coupelles inférieure 8 et supérieure 9, autorise un désaxement des éléments E1, E2 qui peuvent dans une certaine mesure s'orienter indépendamment suivant différentes directions par glissement sur la bille 7 des deux calottes opposées formées par les coupelles 8, 9, grâce à leur diamètre 10 sensiblement supérieur à celui de la portée 4 sur laquelle elles sont enfilées.

Lorsque les éléments E1, E2 sont convenablement orientés, avec l'écart intervertébral approprié, les écrous 6 sont serrés et bloqués élastiquement ce qui empêche tout desserrage ultérieur intempestif.

La configuration de la face 12 des coupelles suivant une surface tronconique, ou éventuellement à facettes, facilite le prépositionnement axial des coupelles ainsi que leur mise en place lors du serrage de l'écrou 6.

L'extrémité supérieure des vis pédiculaires 1, 2 est percée d'un trou borgne dans lequel est conformée une empreinte à six pans creux 28 (figure 2).

Cette empreinte 28 est accessible à travers l'écrou 6 par une clef appropriée afin de bloquer en rotation la vis (1, 2) lors du serrage de l'écrou 6.

La liaison intervertébrale ainsi constituée absorbe élastiquement les chocs et tout en ayant la rigidité adéquate, présente une souplesse suffisante pour autoriser au patient divers mouvements du buste par rapport au bassin, tels que flexions, rotations, ce qui accroît d'autant le confort dudit patient.

La figure 5 illustre une variante de la solidarisation élastique entre la tige filetée 18 et l'élément E2. Suivant cette variante, le logement 20 du bloc 19 est muni sur sa paroi cylindrique d'une gorge circulaire 29 à section en V, susceptible de recevoir des billes de blocage 30 logées à la fois dans la gorge 29 et dans la gorge annulaire 25 de la tête 21, le bloc-tampon élastique 24 étant comprimé.

La gorge 29 est accessible de l'extérieur du bloc 19 par un alésage fileté 31 recevant un petit bloc 32 en matériau élastique occupant la place d'une bille 30 et pressé par une vis 33. Lorsque la tête 21 est en position d'enfoncement correct dans le logement 20, les billes 30 sont introduites par l'alésage 31 dans l'espace annulaire 25-29, puis l'alésage est obturé par les éléments 32, 33. La vis 33 est serrée une fois la tige 18 correctement positionnée dans son manchon 16, pour comprimer le bloc 32 interdisant ainsi efficacement toute rotation ultérieure intempestive de la tige 18 dans son manchon 16.

Aux deux extrémités du dispositif de liaison intervertébrale, les moyens de contention-compression de la bille des deux vis pédiculaires extrêmes peuvent être allégés, puisqu'il n'y a pas de départ d'une autre liaison, en prévoyant la prise en sandwich des billes en question entre la coupelle de l'unique élément de liaison et une coupelle simple faisant office de rondelle interposée entre la bille et la tête hexagonale de la vis pédiculaire ou l'écrou de blocage de l'ensemble, selon le cas.

Cette coupelle simple est identique aux coupelles 8; 9 excepté qu'elle n'est pas rattachée comme ces dernières aux éléments 16, 19.

Une telle coupelle simple est représentée en 40 sur la figure 7 qui illustre une variante du dispositif de l'invention dont une vue en éclaté est représentée en figure 6.

Dans cette variante les vis pédiculaires 1', 2', têtes hexagonales 3', portées 4', billes 7', écrous de blocage 6', coupelles inférieure 8' et supérieure 9', trous de passage 10' sont sensiblement identiques aux éléments homologues du mode de réalisation de la figure 1.

Seul le dispositif intervis de liaison E'1 et E'2 et la tige de jonction 18' diffèrent légèrement de leurs homologues de ladite figure 1.

En effet, l'extrémité filetée 41 de la tige 18' est engagée dans un taraudage d'un manchon 42 introduit dans un trou borgne 43 ménagé dans l'élément E'1.

Le manchon 42 est muni d'une collerette externe 44 à pans coupés et est retenu prisonnier dans le logement 43, tout en pouvant pivoter autour de son axe, grâce à un jeu de billes 45 engagées dans deux rigoles circulaires ménagées en regard dans les parois du manchon 42 et du trou 43 respectivement.

Le blocage en rotation sur lui-même du manchon 42 peut être obtenu à l'aide d'un petit bloc 46 en matériau élastique inséré entre deux billes 45 et introduit par un perçage latéral 47 de l'élément E'1. Ce même perçage 47 est taraudé pour recevoir une vis 48 de pressage du bloc élastique 46 contre le manchon 42, interdisant ainsi sa rotation.

A son autre extrémité la tige 18' comporte une tête cylindrique élargie 49 pourvue, d'une part, d'un trou borgne 50 sur sa face d'extrémité et, d'autre part, de deux méplats latéraux parallèles 51.

La tête 49 est susceptible de s'engager librement dans un trou borgne 52 ménagée dans l'autre élément E'2 en comprimant un petit bloc cylindrique 53 en matériau élastique, introduit au préalable dans le trou 50 et s'interposant entre les fonds des deux trous borgnes 50,52.

La rotation autour de son axe de la tige 18' est interdite à l'aide de deux goupilles 54 engagées dans deux trous parallèles 55 de l'élément E'2 (figure 8) en empiétant dans le trou 52 et en s'engageant dans les espaces dégagés par les méplats 51.

La tige 18' qui est poussée par le bloc comprimé 53 est retenue par les goupilles 54 en contact avec les flancs 56 (figures 6 et 8) des entailles définissant les méplats 51.

L'éloignement entre les éléments E'1, E'2 est réglé en faisant tourner, à l'aide d'une clé plate par exemple, la collerette hexagonale 44 du manchon 42, après desserrage de la vis 48 bien entendu. La rotation du manchon 42 provoque le déplacement axial de la tige 18' dans le manchon 42, puisque la rotation de la tige est interdite par les méplats 51 et les goupilles 54. Par ailleurs, la tige 18' est constamment en butée élastique contre le fond du trou 52 ce qui assure un certain débattement axial de la tige par rapport à l'élément E'2, quel que soit l'éloignement entre les éléments E'1, E'2.

Il est à noter également que l'élément E'1 (figure 6) est muni en partie inférieure de deux prolongements plats 57 en regard susceptibles, lors du montage du dispositif (figure 6'), de se positionner contre deux facettes opposées de la tête hexagonale 3', immobilisant ainsi en rotation mutuelle la vis pédiculaire 1' et la liaison intervis (E'1, 18').

Enfin, l'invention n'est évidemment pas limitée aux modes de réalisation représentés et décrits ci-dessus, mais en couvre au contraire toutes les variantes, notamment en ce qui concerne les nature, forme et dimensions des éléments compressibles élastiquement (7, 7', 24, 32), les formes et dimensions des coupelles 8, 8', 9, 9', ainsi que la nature de la solidarisation élastique entre la tige 18, 18' et son élément porteur E2,E'2 et le moyen de réglage de l'écartement entre les éléments E1, E'1, E2, E'2.

Il est à noter également que selon les applications, les billes 7, 7' peuvent être éventuellement en un matériau rigide.

## Revendications

1. Dispositif de liaison intervertébrale implantable comprenant au moins deux vis pédiculaires (1, 2 ; 1', 2') munies en partie supérieure d'une tête hexagonale (3, 3') prolongée par une portée cylindrique (4, 4') et une liaison intervis de longueur réglable ancrée à chaque extrémité sur lesdites portées, caractérisé en ce que ladite liaison intervis est constituée, d'une part, d'une bille (7, 7') enfilée sur chaque portée (4) et, d'autre part, de deux éléments de liaison (E1, E2 ; E'1, E'2) dans le prolongement l'un de l'autre et reliés entre eux par un système à vis (17, 18 ; 18', 41, 42) de réglage d'éloignement, les extrémités libres des éléments étant conformées en coupelle (8, 9 ; 8', 9') concave embrochable sur ladite portée (4, 4') et épousant ladite bille (7, 7') en sorte qu'au droit de chaque vis pédiculaire (1, 2 ; 1', 2') la bille soit prise en sandwich entre deux coupelles (8, 9 ; 8', 9') de deux liaisons associées à la vis, des moyens (6, 6') étant prévus pour rapprocher l'une vers l'autre les deux coupelles (8, 9 ; 8', 9') en serrant ladite bille (7, 7') et solidariser l'ensemble de la vis pédiculaire.

2. Dispositif suivant la revendication 1, caractérisé en ce que la bille (7,7') est en un matériau déformable élastique.

3. Dispositif suivant la revendication 1 ou 2, caractérisé en ce que ledit système à vis reliant les deux éléments (E1, E2) d'une liaison intervis est constitué d'un manchon taraudé (16) solidaire de l'un des éléments et d'une tige filetée (18) coopérante, reliée à l'autre élément par l'intermédiaire d'un système de blocage à butée élastique (24).

4. Dispositif suivant la revendication 1 ou 2, caractérisé en ce que ledit système à vis reliant les deux éléments (E'1, E'2) d'une liaison intervis est constitué d'une tige (18') munie, à une extrémité, d'une tête filetée (41) engagée dans un taraudage d'un manchon (42) reçu dans l'un (E'1) des deux éléments susdits et à son autre extrémité, d'une tête cylindrique (49) reçue dans l'autre (E'2) des éléments, des moyens étant prévus, d'une part, pour permettre soit la libre rotation, soit le blocage en rotation dudit manchon (42) dans l'élément porteur (E'1) et, d'autre part, pour retenir et bloquer en rotation ladite tête cylindrique (49) dans l'élément porteur (E'2), une butée élastique (53) étant prévue entre la tête et l'élément porteur.

5. Dispositif suivant l'une des revendications 1 à 4, caractérisé en ce que lesdites coupelles (8, 9 ; 8', 9') présentent un trou (10, 10') de passage de la portée cylindrique (4, 4') des vis pédiculaires (1, 2 ; 1', 2'), de diamètre sensiblement supérieur à celui de ladite portée.

6. Dispositif suivant l'une des revendications 1 à 5, caractérisé en ce que lesdits moyens pour rapprocher les coupelles (8, 9 ; 8', 9') et solidariser l'ensemble de la vis pédiculaire (1, 2 ; 1', 2') sont constitués d'un écrou de blocage (6, 6') vissé sur l'extrémité filetée (5) de ladite portée (4, 4').

7. Dispositif suivant la revendication 6, caractérisé en ce que les faces (14, 15), respectivement de la tête hexagonale (3, 3') et de l'écrou (6, 6') de blocage de l'ensemble, tournées vers la bille (7, 7') sont configurées en correspondance avec la face en regard des coupelles (8, 9 ; 8', 9').

8. Dispositif suivant l'une des revendications 1 à 7, caractérisé en ce que la bille (7 ; 7') est sphérique.

9. Dispositif suivant l'une des revendications 1 à 7, caractérisé en ce que la face (12) des coupelles (8, 9 ; 8', 9') tournée vers la bille (7, 7') présente une surface sphérique, tronconique ou à facettes.

10. Dispositif suivant l'une des revendications 3 à 9, caractérisé en ce que ladite tige (18, 18') présente une tête (21, 49) en butée élastique dans un logement (20, 52) dudit élément (E2, E'2), des moyens étant prévus pour retenir et bloquer en rotation ladite tête (21, 49) dans ledit logement (20, 52).

11. Dispositif suivant la revendication 10, caractérisé en ce que lesdits moyens de retenue-blocage de la tête (21, 49) sont constitués par des goupilles (27, 54) de retenue de ladite tête (21, 49) en position de compression d'un bloc (24, 53) en matériau compressible élastique logé dans ledit logement (20, 52).

12. Dispositif suivant la revendication 10, caractérisé en ce que lesdits moyens de retenue-blocage de la tête (21) sont constitués par un jeu de billes (30) interposées entre la tête (21), et son logement (20) et retenant la tête en position de compression d'un bloc (24) en matériau compressible élastique logé dans ledit logement (20), cependant qu'un blocage en rotation de ladite tête (21) est assuré par une vis latérale (33) pressant un bloc (32) en matériau compressible élastique contre ladite tête (21).

13. Dispositif suivant la revendication 10, caractérisé en ce que lesdits moyens de retenue-blocage de la tête cylindrique (49) sont constitués par un jeu de goupilles (54) coopérant avec des méplats (51) ménagées sur ladite tête (49) en sorte de bloquer en rotation ladite tige (18').

14. Dispositif suivant la revendication 3, caractérisé en ce qu'entre la tige filetée (18) et la tête (21) est ménagée une section hexagonale (22) pour assurer la rotation de la tige filetée (18) dans son manchon (16).

15. Dispositif suivant la revendication 4, caractérisé en ce que ledit manchon (42) présente une collerette externe (44) permettant la rotation du manchon dans son logement (43).

16. Dispositif suivant l'une des revendications 1 à 15, caractérisé en ce que l'élément de liaison (E'1) tourné vers la tête hexagonale (3') de la vis pédiculaire (1') comporte deux prolongements inférieurs en regard (57) susceptibles de coopérer avec ladite tête hexagonale (3') à des fins d'immobilisation en rotation de cette dernière.

17. Dispositif suivant l'une des revendications 1 à 16, caractérisé en ce qu'au niveau des deux vis pédiculaires extrêmes de la liaison intervertébrale le serrage de la bille (7') est assuré entre la coupelle (8') de l'élément de liaison (E'2) terminal et une coupelle simple (40) faisant office de rondelle interposée entre la bille et la tête hexagonale (3') de la vis ou l'écrou de blocage (6') de l'ensemble de la vis.
